# FASCICULE DE BREVET EUROPEEN

(11) **EP 1 920 755 B1**
(45) Date de publication et mention de la délivrance du brevet: **29.08.2018**
(21) Numéro de dépôt: 07301536.4
(22) Date de dépôt: 09.11.2007
(51) Int. Cl.: A61K 8/20, A61K 8/36, A61Q 5/04

(54) **Procédé de déformation permanente des fibres kératiniques comprenant une étape d'application d'une composition de rinçage intermédiaire comprenant un sel de cation métallique monovalent ou un sel d'ammonium et un acide organique**
Verfahren zur Dauerwellung von Keratinfasern, das einen Schritt des Auftrags eines Zwischenspülprodukts umfasst, das ein Salz eines einwertigen Metallkations oder ein Salz von Ammonium und einer organischen Säure enthält
Method for permanently deforming keratinous fibres comprising a step of applying an intermediate rinsing component comprising a monovalent metal cation salt or an ammonia salt and an organic acid

(30) Priorité: 10.11.2006 FR 0654853
(43) Date de publication de la demande: 14.05.2008
(73) Titulaire: L'Oréal, 75008 Paris (FR)
(72) Inventeur: Sabbagh, Anne, 92500 RUEIL MALMAISON (FR); Sow, Fatoumata, 92100 CLICHY (FR)
(74) Mandataire: Caillet, Isabelle

(56) Documents cités:
- US-A- 4 134 411
- US-A- 5 942 009

## Description

La présente invention se rapporte à un procédé de déformation permanente des cheveux comprenant une étape d'application d'une composition réductrice, une étape d'application d'une composition de rinçage intermédiaire comprenant au moins un sel monovalent et au moins un acide organique, et une étape d'application d'une composition oxydante, l'application de la composition de rinçage intermédiaire n'étant ni précédée ni suivie d'un rinçage à l'eau.

La technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, généralement à l'eau, à reconstituer dans un second temps lesdites liaisons disulfures en appliquant, sur les cheveux préalablement mis sous tension (bigoudis et autres) ou mis en forme ou lissés par d'autres moyens, une composition oxydante (étape d'oxydation, dite aussi de fixation) de façon à donner finalement aux cheveux la forme recherchée. Cette technique permet ainsi de réaliser indifféremment soit l'ondulation des cheveux (procédé de permanente), soit leur défrisage ou leur décrêpage (procédé de lissage). La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique ou l'acide thioglycolique, leurs sels ainsi que leurs esters, notamment le thioglycolate de glycérol.

Les compositions oxydantes nécessaires à la mise en oeuvre de l'étape de fixation sont le plus souvent des compositions à base d'eau oxygénée.

Cependant, une telle technique ne donne pas entièrement satisfaction. En effet, cette technique est très efficace pour modifier la forme des cheveux, mais très dégradante pour les fibres capillaires.

Ainsi, par exemple, si la technique de déformation permanente des cheveux décrite précédemment est appliquée sur des cheveux ayant fait l'objet au préalable d'une coloration artificielle, elle entraîne une dégradation ou un décapage de cette coloration artificielle.

De même, si une coloration est appliquée sur des cheveux permanentés selon la technique décrite précédemment, la couleur obtenue est très différente de la couleur normalement obtenue sur des cheveux naturels non permanentés.

Par ailleurs, le rinçage à l'eau, entre l'étape de réduction et l'étape d'oxydation, présente également l'inconvénient d'entraîner un surgonflement de la fibre kératinique. Un tel surgonflement altère les propriétés cosmétiques des fibres kératiniques, notamment la douceur, la brillance, le toucher, ainsi que l'aptitude au lissage ou au démêlage.

Enfin, généralement, la composition réductrice présente un pH alcalin obtenu par la présence d'un ou plusieurs agents alcalins. Après l'application de la composition réductrice, on rince généralement abondamment à l'eau pour éliminer toute trace de résidus alcalins. Cela a pour effet d'augmenter la consommation en eau et les rejets dans l'environnement.

On connaît du document US 6,173,717 un procédé de déformation permanente des cheveux qui vise à éviter le bouclage excessif des cheveux et qui comprend l'application sur les cheveux d'une composition réductrice, un temps de pause, un rinçage optionnel à l'eau, l'application d'une composition aqueuse acide intermédiaire comprenant un sel, de préférence divalent, un acide organique aliphatique et une bétaïne, la mise sous tension des cheveux, un nouveau temps de pause, puis l'application d'une composition oxydante.

On connaît également du document EP 443 356 un procédé de déformation permanente des cheveux comprenant une étape de réduction, une étape intermédiaire de rinçage et une étape d'oxydation. L'étape intermédiaire de rinçage est effectuée au moyen d'une solution aqueuse telle qu'à aucun moment pendant le rinçage, le diamètre des fibres n'augmente de plus de 30% par rapport au diamètre des fibres à la fin de l'étape de réduction. En particulier, la solution aqueuse utilisée pour l'étape intermédiaire de rinçage est une solution aqueuse de chlorure de sodium.

On connaît encore du document FR 2 721 823 un procédé de déformation permanente des cheveux comprenant une étape d'application sur les cheveux d'une composition acide contenant un acide carboxylique et/ou l'un de ses sels associés, une étape de rinçage éventuel à l'eau, une étape d'application d'une composition réductrice, la mise sous tension des cheveux étant effectuée avant pendant ou après ladite application de la composition réductrice ou avant l'application de la composition acide, une étape de rinçage à l'eau, puis une étape d'application d'une composition oxydante.

On connaît du document FR 2 718 351 un procédé de déformation permanente des matières kératiniques comprenant une étape d'application d'une composition réductrice, la mise sous tension de la matière kératinique étant effectuée avant pendant ou après ladite application, une étape de rinçage à l'eau, et une étape d'application d'une composition acide comprenant un acide carboxylique, ledit procédé ne comprenant pas d'étape chimique de fixation par oxydation.

On connaît enfin du document EP 0 880 916 un procédé de déformation permanente des cheveux dans lequel on enroule les cheveux sur des rouleaux, on applique sur les cheveux enroulés une composition réductrice, on laisse agir, on traite les boucles, sans rinçage préalable à l'eau, avec une composition de rinçage intermédiaire acide et aqueuse contenant un acide aliphatique organique et un composant de soin et de conditionnement des cheveux, on enlève par tamponnement le liquide éventuellement excédentaire des boucles, on laisse agir, on rince éventuellement avec de l'eau, on traite les cheveux avant et/ou après enlèvement des rouleaux au moyen d'un fixatif à base d'oxydant, et après un temps d'action, on enlève le fixatif des cheveux par rinçage à l'eau, ou par lavage au moyen d'un shampoing. La composition de rinçage intermédiaire peut contenir un sel tel que le cétylstéaryl sulfate de sodium ou le chlorure de diméthyl diallyl ammonium.

Du document US 5 942 009, on connaît un procédé de déformation permanente et de coloration réalisées le même jour. Il comprend l'application de deux compositions intermédiaires particulières après celle de la composition réductrice. Ces compositions intermédiaires contiennent notamment de l'acide citrique et un sel MgSO₄.7H₂O. D'autres sels peuvent être utilisés dans la deuxième composition intermédiaire, comme l'acétate de potassium.

Aucun des procédés décrits ne conduit à un procédé de déformation permanente totalement satisfaisant vis-à-vis des problèmes mentionnés ci-dessus.

Il existe donc un besoin de disposer d'un procédé de déformation permanente des fibres kératiniques qui ne dégrade pas la coloration artificielle des cheveux et qui n'altère pas les propriétés cosmétiques, telles que la douceur, la brillance, le toucher, ainsi que l'aptitude au lissage et au démêlage.

La demanderesse a découvert que les problèmes de l'art antérieur étaient résolu par un procédé de déformation permanente des fibres kératiniques comprenant une étape de réduction, les fibres kératiniques étant mises sous tension mécanique avant, pendant ou après ladite étape de réduction, puis une étape de rinçage intermédiaire au moyen d'une composition comprenant au moins un sel de cation métallique monovalent et/ou au moins un sel d'ammonium, le ou les sels étant choisis parmi NaCl, NH4Cl, KCl et AgCl, et au moins un acide organique, le ou lesdits sels n'étant pas un ou des sels du ou desdits acides organiques présents dans la composition de rinçage intermédiaire, et le ou lesdits sels n'étant pas un ou des tensioactifs, puis une étape d'oxydation, ledit procédé ne comprenant pas d'étape de rinçage à l'eau entre l'étape de réduction et l'étape de rinçage intermédiaire, ni entre l'étape de rinçage intermédiaire et l'étape d'oxydation.

L'invention a donc pour objet un procédé de déformation permanente des fibres kératiniques, en particulier des cheveux, comprenant :
- une étape d'application sur les fibres kératiniques d'une composition réductrice, pour réduire les liaisons disulfure de la kératine, les fibres kératiniques étant mises sous tension mécanique avant, pendant ou après ladite application, de préférence avant, puis
- une étape d'application sur les fibres kératiniques d'une composition de rinçage intermédiaire comprenant au moins un sel de cation métallique monovalent et/ou au moins un sel d'ammonium, le ou les sels étant choisis parmi NaCl, NH4Cl, KCl et AgCl, et au moins un acide organique, le ou lesdits sels monovalents n'étant pas un ou des sels du ou desdits acides organiques présents dans la composition de rinçage intermédiaire, et le ou lesdits sels n'étant pas un ou des tensioactifs, puis
- une étape de fixation par oxydation, pour reformer lesdites liaisons, par application sur les fibres kératiniques d'une composition oxydante,
ledit procédé ne comprenant pas d'étape de rinçage à l'eau entre l'étape d'application de la composition réductrice et l'étape d'application de la composition de rinçage intermédiaire, ni entre l'étape d'application de la composition de rinçage intermédiaire et l'étape d'application de la composition oxydante.

Selon un mode de réalisation préféré, on laisse la composition réductrice agir pendant 5 à 60 minutes, de préférence pendant 5 à 30 minutes.

La composition réductrice utilisée dans le procédé selon l'invention comprend généralement, dans un milieu cosmétiquement acceptable, au moins un agent réducteur choisi parmi les agents réducteurs de formule

H(X')_{q}(R')ᵣ

dans laquelle X' représente P, S ou SO₂, q vaut 0 ou 1, r vaut 1 ou 2 et R' est un radical (C₁-C₂₀)hydrocarboné, linéaire, ramifié, saturé insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁-C₃₀)alkyl)amino carbonyle, un groupement (C₁-C₃₀)acyle) amino, un groupement mono ou dialkylamino, un groupement mono ou dihydroxylamino,
ou l'un de ses sels en combinaison avec une base.

De préférence, le ou les agents réducteurs sont choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

Le ou les agents réducteurs représentent généralement de 0,05 à 30%, de préférence de 1 à 20%, mieux de 4 à 11% en poids par rapport au poids total de la composition réductrice.

Dans le but d'améliorer les propriétés cosmétiques des fibres capillaires ou encore d'atténuer ou d'éviter leur dégradation, la composition réductrice utilisée dans le procédé selon l'invention peut également comprendre un ou plusieurs actifs cosmétiques.

Ce ou ces actifs sont généralement choisis parmi les silicones volatiles ou non, linéaires ou cycliques, aminées ou non, les polymères cationiques, anioniques, non ioniques ou amphotères, les peptides et leurs dérivés, les hydrolysats de protéines, les corps gras, les cires naturelles ou synthétiques, et en particulier les alcools gras, les agents de gonflement et de pénétration ou permettant de renforcer l'efficacité du ou des réducteurs, ainsi que d'autres composés actifs tels que les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents chélatants, les agents opacifiants, les colorants, les filtres solaires, les vitamines ou provitamines, les huiles minérales, végétales ou synthétiques, ainsi que les parfums et les conservateurs, et leurs mélanges.

A titre d'agents chélatants, on peut citer par exemple le sel pentasodique de l'acide diéthylène triamine pentaacétique.

A titre d'agents tensioactifs, on peut citer par exemple l'alcool oléique oxyéthyléné à 20 moles d'oxyde d'éthylène, le mélange cocoyl amidopropryl bétaine/mono-laurate de glycéryle, l'oxyde de lauryl diméthyl amine, le chlorure de béhényl triméthyl ammonium, l'alcool stéarylique, le mono distéarate de glycéryle, le monoéthanolamide d'acide alkyl (C13/C15 70/30 50% linéaire) éther carboxylique (20E), le mélange alcool cétylstéarylique/alcool cétylstéarylique oxyéthylène (30 OE) et le chlorure d'oléocétyl diméthyl hydroxyéthyl ammonium.

A titre de conservateur, on peut citer par exemple le salicylate de sodium.

A titre d'opacifiant, on peut citer par exemple le copolymère vinylpyrrolidone/styrène.

A titre de corps gras, on peut citer par exemple la vaseline.

A titre d'épaississant, on peut citer l'alcool oléique et l'alcool cétylique.

Au sens de la présente invention, l'expression "polymère cationique" désigne tout polymère contenant des groupements cationiques et/ou des groupements ionisables en groupements cationiques.

Les polymères cationiques éventuellement présents dans la composition réductrice utilisée dans le procédé selon l'invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n°2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 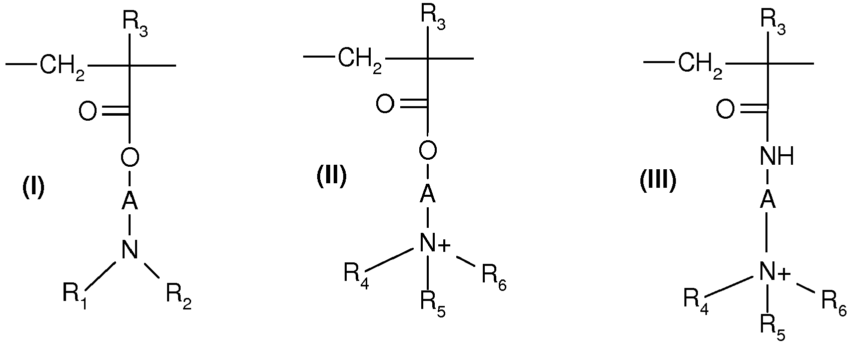 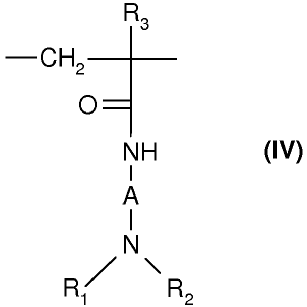 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   On peut citer en particulier l'homopolymère chlorure de méthacrylate d'éthyl triméthyl ammonium.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyl-triméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxy-éthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthyl-aminopropyle quaternisés tel que le produit vendu sous la dénomination "GAFQUAT HS 100" par la société ISP .
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 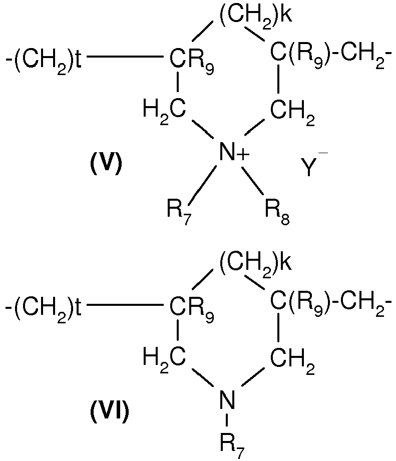 formules dans lesquelles k et t sont égaux à 0 ou 1, la somme k + t étant égale à 1 ; Rg désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 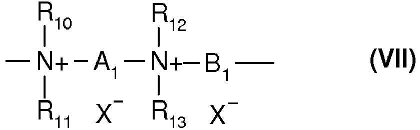 formule (VII) dans laquelle :
   R₁₀, R_{11,} R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R_{11,} R₁₂ et R₁₃, ensemble ou séparément, constituent avec les atomes d'azote auxquels ils sont rattachés des hétérocycles contenant éventuellement un second hétéroatome autre que l'azote ou bien R₁₀, R_{11,} R₁₂ et R₁₃ représentent un radical alkyle en C₁-C₆ linéaire ou ramifié substitué par un groupement nitrile, ester, acyle, amide ou -CO-O-R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 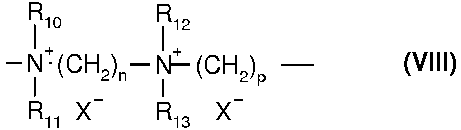 dans laquelle R₁₀, R_{11,} R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique. On peut citer en particulier le MEXOMERE PO commercialisé par la société CHIMEX.
(11) Les polyammoniums quaternaires constitués de motifs récurrents de formule (IX) : 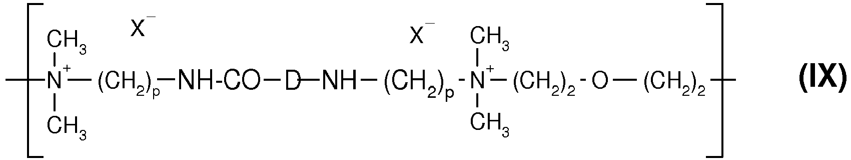 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA, ou les polyamines de coprah oxyéthyléné (15 OE).
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 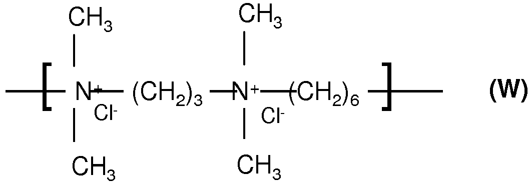 et notamment ceux dont le poids moléculaire, déterminé par chromatographie par perméation de gel, est compris entre 9500 et 9900; 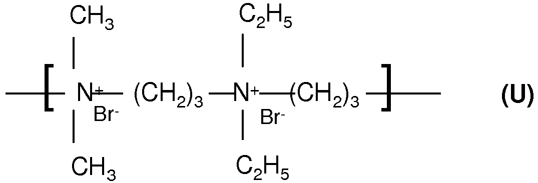 et notamment ceux dont le poids moléculaire, déterminé par chromatographie par perméation de gel, est d'environ 1200.

La concentration en polymère cationique dans la composition réductrice utilisée dans le procédé selon l'invention peut varier de 0,01 à 10% en poids par rapport au poids total de la composition, de préférence de 0,05 à 5% et plus préférentiellement encore de 0,1 à 3%.

Les polymères amphotères éventuellement présents dans la composition réductrice utilisée dans le procédé selon l'invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes ;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'amino-éthyle, de butyl aminoéthyle, de N,N'-diméthylaminoéthyle, de N-tertio-butyl-aminoéthyle.
   On utilise particulièrement les copolymères dont la dénomination CTFA (4ème Ed., 1991) est Octylacrylamide/acrylates/butyl-aminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ **(X)**

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 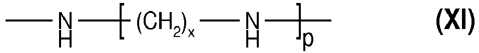 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 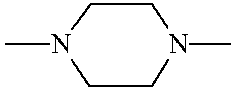
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 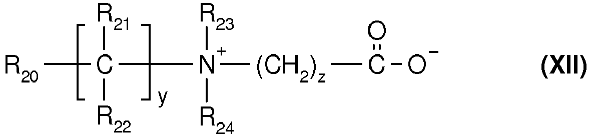 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl-carboxyméthylammonio-éthylméthacrylate de méthyle tel que le produit vendu sous la dénomination DIAFORMER Z301 par la société SANDOZ.
(5) les polymères dérivés du chitosane comportant des motifs monomères répondant aux formules (XIII), (XIV), (XV) suivantes : 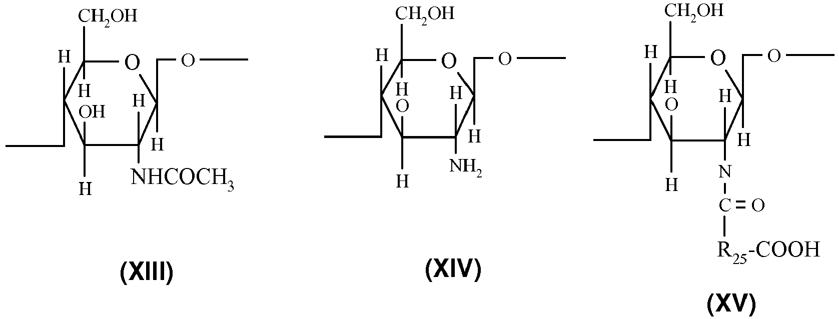 le motif (XIII) étant présent dans des proportions comprises entre 0 et 30%, le motif (XIV) dans des proportions comprises entre 5 et 50% et le motif F dans des proportions comprises entre 30 et 90%, étant entendu que dans ce motif (XV), R₂₅ représente un radical de formule : 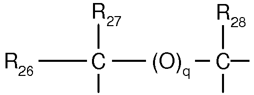 dans laquelle
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 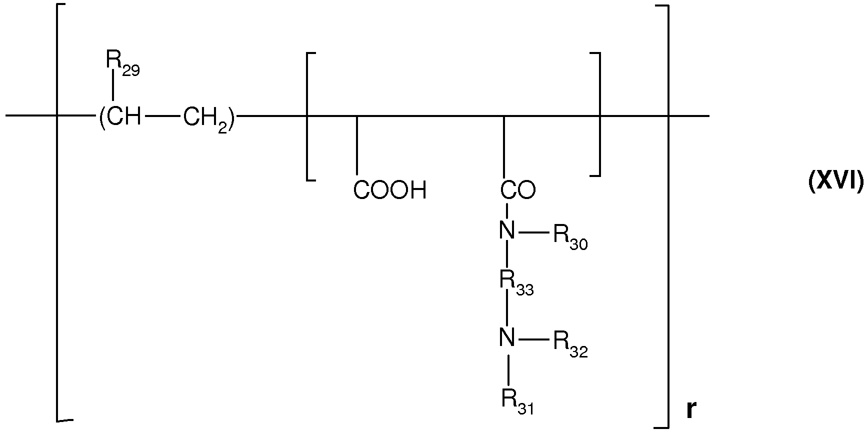 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus, ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 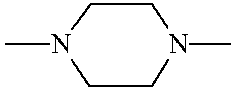 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)

      où D désigne un radical 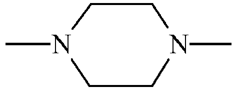 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition réductrice.

Les polymères anioniques éventuellement présents dans la composition réductrice utilisée dans le procédé selon l'invention sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 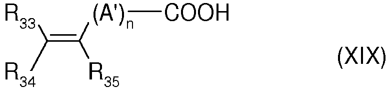 dans laquelle n est un nombre entier de 0 à 10, A' désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₃₃ désigne un atome d'hydrogène, un groupement phényle ou benzyle, R₃₄ désigne un atome d'hydrogène, un groupement alkyle inférieur ou carboxyle, R₃₅ désigne un atome d'hydrogène, un groupement alkyle inférieur, un groupement -CH₂-COOH, phényle ou benzyle.

Dans la formule (XIX) ci-dessus, un groupement alkyle inférieur comporte de préférence de 1 à 4 atomes de carbone et désigne en particulier, les groupements méthyle et éthyle.

Les polymères anioniques à groupements carboxyliques préférés selon l'invention sont :
A) les homo- ou copolymères d'acide acrylique ou méthacrylique ou leurs sels, et en particulier les produits vendus sous les dénominations VERSICOL® E ou K par la société ALLIED COLLOID, ULTRAHOLD® par la société BASF Les copolymères d'acide acrylique et d'acrylamide vendus sous la forme de leur sel de sodium sous les dénominations RETEN® 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER® par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER® MAEX par la société BASF.
C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
- les sels de l'acide polystyrène-sulfonique, les sels de sodium, ayant une masse moléculaire d'environ 500 000 et d'environ 100 000 vendus respectivement sous les dénominations Flexan® 500 et Flexan® 130 par National Starch. Ces composés sont décrits dans le brevet FR 2198719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER® HSP 1180 par Henkel.

Les polymères non-ioniques éventuellement présents dans la composition réductrice utilisée dans le procédé selon l'invention peuvent être choisis parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX® 50 000, PEOX® 200 000 et PEOX® 500 000 ;
- les homopolymères d'acétate de vinyle tels que le produit proposé sous le nom de APPRETAN® EM par la société HOECHST ou le produit proposé sous le nom de RHODOPAS® A 012 par la société RHONE POULENC ;
- les copolymères d'acétate de vinyle et d'ester acrylique tels que le produit proposé sous le nom de RHODOPAS® AD 310 de RHONE POULENC ;
- les copolymères d'acétate de vinyle et d'éthylène comme le produit proposé sous le nom de APPRETAN® TV par la société HOECHST;
- les copolymères d'acétate de vinyle et d'ester maléique, par exemple de maléate de dibutyle tels que le produit proposé sous le nom de APPRETAN® MB EXTRA par la société HOECHST ;
- les copolymères de polyéthylène et d'anhydride maléique ;
- les homopolymères d'acrylates d'alkyle et les homopolymères de méthacrylates d'alkyle tels que le produit proposé sous la dénomination MICROPEARL® RQ 750 par la société MATSUMOTO ou le produit proposé sous la dénomination LUHYDRAN® A 848 S par la société BASF ;
- les copolymères d'esters acryliques tels que, par exemple, les copolymères d'acrylates d'alkyle et de méthacrylates d'alkyle comme les produits proposés par la société ROHM & HAAS sous les dénominations PRIMAL® AC-261 K et EUDRAGIT® NE 30 D, par la société BASF sous les dénominations ACRONAL® 601, LUHYDRAN® LR 8833 ou 8845, par la société HOECHST sous les dénominations APPRETAN® N 9213 ou N921 2 ;
- les copolymères d'acrylonitrile et d'un monomère non ionique choisi, par exemple, parmi le butadiène et les (méth)acrylates d'alkyle; on peut citer les produits proposés sous les dénominations NIPOL® LX 531 8 par la société NIPPON ZEON ou ceux proposés sous la dénomination CJ 0601 8 par la société ROHM & HAAS ;
- les polyuréthannes tels que les produits proposés sous les dénominations ACRYSOL® RM 1020 ou ACRYSOL® RM 2020 par la société ROHM & HAAS, les produits URAFLEX® XP 401 UZ, URAFLEX® XP 402 UZ par la société DSM RESINS ;
- les copolymères d'acétate d'alkyle et d'uréthanne tels que le produit 8538-33 par la société NATIONAL STARCH ;
- les polyamides tels que le produit ESTAPOR® LO 11 proposé par la société RHONE POULENC ;
- les gommes de guar non ioniques chimiquement modifiées ou non modifiées.

Les gommes de guar non ioniques non modifiées sont, par exemple, les produits vendus sous la dénomination VIDOGUM® GH 175 par la société UNIPECTINE et sous la dénomination JAGUAR® C par la société MEYHALL.

Les gommes de guar non-ioniques modifiées, utilisables selon l'invention, sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont, par exemple, vendues sous les dénominations commerciales JAGUAR® HP8, JAGUAR® HP60 et JAGUAR® HP120, JAGUAR® DC 293 et JAGUAR® HP 105 par la société MEYHALL, ou sous la dénomination GALACTASOL® 4H4FD2 par la société AQUALON.

Les groupes alkyle des polymères non ioniques comportent de préférence de 1 à 6 atomes de carbone.

La composition réductrice utilisée dans le procédé selon l'invention peut également contenir une ou plusieurs silicones.

Les silicones éventuellement présentes dans la composition réductrice utilisée dans le procédé selon l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.

Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.

On peut également citer les cyclocopolymères du type diméthylsiloxanes/ méthylalkylsiloxane, tel que la "SILICONE VOLATILE FZ 3109" commercialisée par la société UNION CARBIDE, de structure chimique :

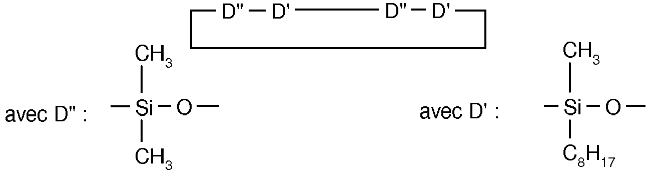

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

On peut également citer les polydiméthylsiloxanes à groupements aminoéthyl aminopropyl et alpha-oméga silanols.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
• les huiles SILBIONE de la série 70 641 de RHONE POULENC ;
• les huiles des séries RHODORSIL 70 633 et 763 de RHONE POULENC ;
• l'huile DOW CORNING 556 COSMETIC GRAD FLUID de DOW CORNING ;
• les silicones de la série PK de BAYER comme le produit PK20 ;
• les silicones des séries PN, PH de BAYER comme les produits PN1000 et PH1000 ;
• certaines huiles des séries SF de GENERAL ELECTRIC telles que SF 1023, SF 1154, SF 1250, SF 1265.

Les gommes de silicone pouvant être présentes dans la composition réductrice du procédé selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes éventuellement présentes dans la composition réductrice du procédé selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition réductrice du procédé selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ; on peut citer les silicones aminées comportant des groupements alcoxy telle que la silicone BELSIL AOM LOG 1 commercialisé par la société WACKER ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

A titre de silicone non ionique, on peut citer le polymère block poly diméthylsiloxane/aminopolyéther (80/20).

Comme expliqué précédemment, le procédé selon l'invention comprend une étape d'application d'une composition de rinçage intermédiaire comprenant au moins un sel de cation métallique monovalent et/ou au moins un sel d'ammonium, le ou les sels étant choisis parmi NaCl, NH4Cl, KCl et AgCl, et au moins un acide organique, le ou lesdits sels monovalents n'étant pas un ou des sels du ou desdits acides organiques présents dans la composition de rinçage intermédiaire, et le ou les sels n'étant pas un ou des tensioactifs.

Par sel n'étant pas un tensioactif, on entend au sens de la présente invention un sel qui ne contient pas une chaîne grasse aliphatique d'au moins 10 atomes de carbone.

Les sels compris dans la composition de rinçage intermédiaire utilisable dans le procédé selon l'invention sont le chlorure de sodium NaCl, le chlorure d'ammonium NH₄Cl, le chlorure de potassium KCI et le chlorure d'argent AgCl.

Le ou les sels compris dans la composition de rinçage intermédiaire utilisable dans le procédé selon l'invention peuvent éventuellement être hydratés.

Généralement, le ou les sels compris dans la composition de rinçage intermédiaire représentent de 0,1 à 20%, de préférence de 0,1 à 10%, mieux de 0,5 à 8% en poids par rapport au poids total de la composition de rinçage intermédiaire.

Le ou les acides organiques présents dans la composition de rinçage intermédiaire sont généralement choisis parmi les acides comportant une ou plusieurs fonctions acide carboxylique, sulfonique, phosphonique ou phosphorique. Ils peuvent contenir d'autres fonctions chimiques en particulier des fonctions hydroxy ou amino. Ils peuvent être saturés ou insaturés.

On peut citer en particulier l'acide acétique, l'acide propanoïque, l'acide butanoïque, l'acide lactique, l'acide glycolique, l'acide ascorbique, l'acide maléique, l'acide phtalique, l'acide succinique, la taurine et l'acide citrique.

Un acide préféré est l'acide citrique.

Le ou les acides organiques représentent généralement de 0,005 à 10%, de préférence de 0,01 à 1% en poids du poids total de la composition de rinçage intermédiaire.

Dans le but d'améliorer les propriétés cosmétiques des fibres capillaires ou encore d'atténuer ou d'éviter leur dégradation, la composition de rinçage intermédiaire utilisée dans le procédé selon l'invention peut également comprendre un ou plusieurs actifs cosmétiques, tels que ceux décrits pour la composition réductrice.

Selon un mode de réalisation particulier de l'invention, la composition de rinçage intermédiaire utilisée dans le procédé selon l'invention comprend un ou plusieurs polymères cationiques, anioniques, non-ioniques ou amphotères, et/ou une ou plusieurs silicones.

De préférence, la composition de rinçage intermédiaire ne comprend pas de tensioactif amphotère.

De préférence encore, la composition de rinçage intermédiaire ne comprend pas de bétaïne.

Le pH de la composition de rinçage intermédiaire est généralement compris entre 2 et 7, de préférence entre 3 et 5.

De préférence, on laisse la composition de rinçage intermédiaire agir pendant 30 secondes à 30 minutes.

Comme expliqué précédemment, le procédé selon l'invention comprend une étape d'application d'une composition oxydante.

La composition oxydante comprend généralement au moins un agent oxydant choisi parmi le peroxyde d'hydrogène, les bromates alcalins, les polythionates, les persels, tels que les perborates, les percarbonates et les persulfates.

De préférence, l'agent oxydant est l'eau oxygénée.

Le ou les agents oxydants représentent généralement de 0,1 à 8%, de préférence de 0,2 à 5%, en poids par rapport au poids total de la composition oxydante.

De préférence, lorsque l'agent oxydant est du peroxyde d'hydrogène en solution aqueuse, la composition oxydante utilisée dans le procédé selon l'invention contient au moins un agent stabilisant de l'eau oxygénée.

On peut citer particulier les pyrophosphates des métaux alcalins ou alcalino-terreux, tel que le pyrophosphate de tétrasodium, les stannates des métaux alcalins ou alcalino-terreux, la phénacétine ou les sels d'acides et d'oxyquinoléine, comme le sulfate d'oxyquinoléine (sulfate de 8-hydroxyquinoléine). De manière plus avantageuse encore, on utilise au moins un stannate en association ou non à au moins un pyrophosphate.

Le ou les agents stabilisants de l'eau oxygénée représentent généralement de 0,0001% à 5% en poids et de préférence de 0,01 à 2% en poids par rapport au poids total de la composition oxydante.

Dans le but d'améliorer les propriétés cosmétiques des fibres capillaires ou encore d'atténuer ou d'éviter leur dégradation, la composition oxydante utilisée dans le procédé selon l'invention peut également comprendre un ou plusieurs actifs cosmétiques, tels que ceux mentionnés précédemment au sujet de la composition réductrice.

Généralement, le pH de la composition oxydante varie de 1 à 13, de préférence de 1,5 à 8, mieux de 1,5 à 5.

Le véhicule des compositions réductrice, de rinçage intermédiaire et oxydante utilisées dans le procédé selon l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, le glycérol, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants organiques peuvent alors être présents dans des concentrations comprises entre environ 0,1 et 20% et, de préférence, entre environ 1 et 10% en poids par rapport au poids total de la composition.

Les pH de la composition réductrice et de la composition oxydante utilisées dans le procédé selon l'invention peuvent être obtenus et/ou ajustés classiquement par ajout soit d'agents alcalins, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique, l'acide citrique et l'acide phosphorique.

La composition réductrice, la composition de rinçage intermédiaire et la composition oxydante utilisées dans le procédé selon l'invention peuvent se présenter indépendamment des autres sous forme de lotion, de gel, de mousse, de crème ou de pâte.

Comme expliqué précédemment, le procédé selon l'invention comprend une étape d'application d'une composition réductrice sur les fibres kératiniques, en particulier sur les cheveux, une étape d'application d'une composition de rinçage intermédiaire, puis une étape d'application d'une composition oxydante.

Selon un mode de réalisation particulier de l'invention, l'étape d'application de la composition réductrice et/ou l'étape d'application de la composition de rinçage intermédiaire et/ou l'étape de fixation s'effectue(nt) sous chauffage ou est (sont) immédiatement suivie(s) d'un chauffage.

Généralement, le procédé selon l'invention comprend, après l'étape de fixation par oxydation, une étape de rinçage des fibres kératiniques à l'eau et/ou au moyen d'un reliquat de la composition de rinçage intermédiaire.

Lorsque l'on souhaite réaliser une permanente, on utilise de préférence des moyens mécaniques tels que des bigoudis, la composition réductrice étant appliquée avant, pendant ou après les moyens de mise en forme des cheveux, de préférence après.

De préférence, on applique la composition réductrice sur des cheveux mouillés préalablement enroulés sur des rouleaux ayant de 2 à 30 mm de diamètre. La composition peut également être appliquée au fur et à mesure de l'enroulage des cheveux. Généralement, on laisse ensuite agir la composition réductrice pendant un temps de 5 à 60 minutes, de préférence pendant 5 à 30 minutes.

On peut également, après application de la composition réductrice, soumettre la chevelure à un traitement thermique par chauffage à une température comprise entre 30 et 250°C pendant tout ou partie du temps de pose. Dans la pratique, cette opération peut être conduite au moyen d'un casque de coiffure, d'un sèche-cheveux, d'un fer rond ou d'un fer plat, d'un dispensateur de rayons infrarouges et d'autres appareils chauffants.

On peut notamment utiliser, à la fois comme moyen de chauffage et de mise en forme de la chevelure, un fer chauffant à une température comprise entre 60 et 220°C et de préférence entre 120 et 200°C, l'utilisation du fer chauffant se faisant entre l'étape d'application de la composition réductrice et l'étape d'application de la composition de rinçage intermédiaire.

Le bigoudi lui-même peut-être chauffant.

On applique alors sur les cheveux la composition de rinçage intermédiaire définie précédemment. On peut laisser poser cette composition pendant 30 secondes à 30 minutes.

On applique ensuite, sur les cheveux enroulés ou déroulés, la composition oxydante permettant de reformer les liaisons disulfures de la kératine, généralement pendant un temps de pose de 2 à 15 minutes. Après avoir enlevé les rouleaux, on rince généralement abondamment la chevelure, généralement à l'eau. On peut éventuellement remplacer le rinçage à l'eau par un rinçage avec le reliquat de la composition de rinçage intermédiaire, ou bien faire suivre le rinçage à l'eau d'un rinçage avec le reliquat de la composition de rinçage intermédiaire.

Lorsque l'on souhaite effectuer le défrisage ou le décrêpage des cheveux, on applique sur les cheveux la composition réductrice, puis l'on soumet les cheveux à une déformation mécanique permettant de les fixer dans leur nouvelle forme, par une opération de lissage des cheveux avec un peigne à larges dents, avec le dos d'un peigne, à la main ou au pinceau. On effectue généralement un temps de pose de 5 à 60 minutes, de préférence de 15 à 45 minutes.

Le lissage des cheveux peut également être effectué, en tout ou partie, à l'aide d'un fer chauffant entre 60 et 220°C, et de préférence entre 120 et 200 °C.

On applique ensuite sur les cheveux la composition de rinçage intermédiaire définie précédemment. On peut également faire suivre cette application d'un traitement chauffant, notamment à l'aide d'un fer.

Puis on applique la composition oxydante telle que définie ci-dessus, qu'on laisse généralement agir pendant environ 2 à 15 minutes, puis on rince généralement abondamment les cheveux, généralement à l'eau et/ou en utilisant le reliquat de la composition de rinçage intermédiaire.

Le procédé de déformation permanente selon l'invention, notamment dans le cas d'un rinçage à pH 3, présente, par rapport à un procédé comprenant une simple étape de rinçage à l'eau entre l'étape de réduction et l'étape d'oxydation, un effet protecteur de la couleur artificielle des cheveux, tant lors de la mise en oeuvre de ce procédé qu'ultérieurement après plusieurs lavages par shampoings.

De même, l'ondulation des cheveux obtenue lors de la déformation permanente des cheveux par le procédé selon l'invention est meilleure, ainsi que le décollement des racines.

Enfin, la présence dans le procédé selon l'invention d'une étape d'application d'une composition de rinçage intermédiaire selon l'invention améliore également les performances cosmétiques, telles que la brillance, le toucher lisse et la facilité de démêlage des cheveux.

L'invention est illustrée par les exemples qui suivent.

### Exemple 1

On prépare une composition réductrice, une composition de rinçage intermédiaire et une composition oxydante pour la mise en oeuvre d'un procédé de déformation permanente des cheveux selon l'invention.

Les formulations sont les suivantes :

### 1. Composition réductrice :

- Acide thioglycolique 6,0%
- Bicarbonate d'ammonium 4,5%
- Mexomere PO 1,7%
- Alcool oléique oxyéthyléné (20 moles d'oxyde d'éthylène) 1,2%
- Mélange cocoyl amidopropyl bétaine/mono-laurate de glycéryle en solution aqueuse à 30% 1,4%
- Polydiméthylsiloxane à groupements aminoéthyl aminopropyl et alpha-oméga silanols en émulsion aqueuse cationique 2%
- Acide diéthylène triamine pentacétique, sel pentasodique en solution aqueuse à 40% 0,2%
- Parfum 0,6%
- Eau déminéralisée qsp 100g%

### 2. Composition de rinçage intermédiaire

| | | |
|---|---|---|
| - Chlorure de sodium | 5 g | |
| - Acide citrique | 0,04 g | pH 3 |
| - Eau déminéralisée | qsp 100 g | |

### 3. Composition fixatrice

- Peroxyde d'hydrogène en solution aqueuse à 50%
   (eau oxygénée à 200 vol) 4,8%
- Sulfate de 8-hydroxyquinoléine 0,0125%
- Salicylate de sodium 0,01%
- Chlorure de poly di-méthyl di allyl ammonium dans l'eau
   à 40% non stabilisée 1,75%
- Oxyde de lauryl diméthyl amine en solution aqueuse 2,15%
- Copolymère vinylpyrrolidone/styrène en émulsion aqueuse
   anionique 0,1%
- Extrait hydroglycolique de citron titre en alpha-hydroxyacides
   protégé 0,1%
- Parfum 0,4%
- Acide citrique qsp pH 3
- Eau déminéralisée qsp 100g%

La composition réductrice est appliquée sur des cheveux humides, préalablement enroulés sur bigoudis. La composition réductrice est laissée en contact avec la chevelure pendant 10 minutes.

On applique alors sur les cheveux la composition de rinçage intermédiaire.

Puis on applique sur les cheveux la composition oxydante. On laisse poser pendant 5 minutes avant de rincer à l'eau et/ou avec le reliquat de la composition de rinçage intermédiaire.

### Exemple 2

On prépare une composition réductrice (crème de lissage), une composition de rinçage intermédiaire et une composition oxydante pour la mise en oeuvre d'un procédé de déformation permanente des cheveux selon l'invention.

Les formulations sont les suivantes :

### Crème de lissage :

- Acide thioglycolique 5,1%
- Dithiodiglycolate de diammonium 2,5%
- Monoéthanolamine 1,0%
- Bicarbonate d'ammonium 2,5%
- Chlorure de béhényl triméthyl ammonium en solution
   dans un mélange eau/isopropanol 2,0%
- Glycérol 2,0%
- Polyamine de coprah oxyéthyléné (150E) en solution
   aqueuse 1,6%
- Alcool oléique 3,0%
- Alcool cétylique 6,0%
- Lanoline 1,0%
- Alcool stéarylique 2,5%
- Mono distéarate de glycéryle 2,0%
- Vaseline 6,0%
- Acide diéthylène triamine pentacétique, sel pentasodique
   en solution aqueuse à 40% 0,4%
- Parfum 1%
- Eau déminéralisée qsp 100g%

### Composition de rinçage intermédiaire

| | | |
|---|---|---|
| - Chlorure de sodium | 5 g | |
| - Acide citrique | 0,04 g | pH 3 |
| - Eau déminéralisée | qsp 100 g | |

### Composition fixatrice

- Peroxyde d'hydrogène en solution aqueuse à 50%
   (eau oxygénée à 200 vol) 4,8%
- Monoéthanolamide d'acide alkyl (C13/C15 70/30 50 % linéaire)
   éther carboxylique (2 OE) 0,6%
- Stannate de sodium, 6 H₂O 0,04%
- Pyrophosphate tétrasodique, 10 H₂O 0,02%
- Homopolymère chlorure de méthacrylate d'éthyl triméthyl
   ammonium réticulé en émulsion inverse dans
   huile minérale 0,75%
- Glycérol 1,5%
- Polymère block poly diméthylsiloxane/aminopolyéther (80/20)
   en solution à 30% dans le dipropylène glycol 1,5%
- Acide phosphorique 0,2%
- Mélange alcool cétylstéarylique/alcool
   cétylstéarylique oxyéthylène (30 OE) 2,0%
- Chlorure d'oléocétyl diméthyl hydroxyéthyl
   ammonium en solution aqueuse 1,0%
- Parfum 0,4%
- Eau déminéralisée qsp 100g%

La crème de lissage est appliquée sur des cheveux humides et propres, puis laissée en contact avec la chevelure pendant 15 minutes.

On applique ensuite sur les cheveux la composition de rinçage intermédiaire.

La composition oxydante est ensuite appliquée, puis laissée en contact avec les cheveux de façon à fixer la forme donnée.

Les cheveux sont ensuite rincés à l'eau et/ou avec le reliquat de la composition de rinçage intermédiaire.

### Exemple 3

Dans les exemples 1 et 2, le chlorure de sodium peut être remplacé par le chlorure d'ammonium. Les résultats obtenus sont similaires.

## Revendications

1. Procédé de déformation permanente des fibres kératiniques, en particulier des cheveux, comprenant :
- une étape d'application sur les fibres kératiniques d'une composition réductrice, pour réduire les liaisons disulfure de la kératine, les fibres kératiniques étant mises sous tension mécanique avant, pendant ou après ladite application, de préférence avant, puis
- une étape d'application sur les fibres kératiniques d'une composition de rinçage intermédiaire comprenant :
- au moins un sel de cation, métallique monovalent et/ou au moins un sel d'ammonium, le ou les sels étant choisis parmi NaCl, NH₄Cl, KCl, et AgCl et
- au moins un acide organique,
le ou lesdits sels n'étant pas un ou des sels du ou desdits acides organiques présents dans la composition de rinçage intermédiaire, et le ou lesdits sels n'étant pas un ou des tensioactifs, puis
- une étape de fixation par oxydation, pour reformer lesdites liaisons, par application sur les fibres kératiniques d'une composition oxydante,
ledit procédé ne comprenant pas d'étape de rinçage à l'eau entre l'étape d'application de la composition réductrice et l'étape d'application de la composition de rinçage intermédiaire, ni entre l'étape d'application de la composition de rinçage intermédiaire et l'étape d'application de la composition oxydante.

2. Procédé selon la revendication 1 **caractérisé en ce que** on laisse la composition réductrice agir pendant 5 à 60 minutes, de préférence pendant 5 à 30 minutes.

3. Procédé selon la revendication 1 ou 2 **caractérisé en ce que** la composition réductrice comprend, dans un milieu cosmétiquement acceptable, au moins un agent réducteur choisi parmi les agents réducteurs de formule
H(X')_{q}(R')ᵣ
dans laquelle X' représente P, S ou SO₂, q vaut 0 ou 1, r vaut 1 ou 2 et R' est un radical (C₁-C₂₀)hydrocarboné, linéaire, ramifié, saturé insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁-C₃₀)alkyl)amino carbonyle, un groupement (C₁-C₃₀)acyle)amino, un groupement mono ou dialkylamino, un groupement mono ou dihydroxylamino, ou l'un de ses sels en combinaison avec une base.

4. Procédé selon la revendication 3 **caractérisé en ce que** le ou les agents réducteurs sont choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

5. Procédé selon la revendication 3 ou 4 **caractérisé en ce que** le ou les agents réducteurs représentent de 0,05 à 30%, de préférence de 1 à 20%, mieux de 4 à 11% en poids par rapport au poids total de la composition réductrice.

6. Procédé selon l'une quelconque des revendications précédentes **caractérisée en ce que** le ou les sels représentent de 0,1 à 20%, de préférence de 0,1 à 10%, mieux de 0,5 à 8% en poids par rapport au poids total de la composition de rinçage intermédiaire.

7. Procédé selon l'une quelconque des revendications précédentes **caractérisé en ce que** le ou les acides organiques sont choisis parmi les acides saturés ou insaturés comportant une ou plusieurs fonctions acide carboxylique, sulfonique, phosphonique ou phosphorique.

8. Procédé selon la revendication 7 **caractérisé en ce que** le ou les acides organiques sont choisis parmi l'acide acétique, l'acide propanoïque, l'acide butanoïque, l'acide lactique, l'acide glycolique, l'acide ascorbique, l'acide phtalique, l'acide maléique, l'acide succinique et l'acide citrique, de préférence l'acide citrique.

9. Procédé selon l'une quelconque des revendications précédentes **caractérisé en ce que** le ou les acides organiques représentent de 0,005 à 10%, de préférence de 0,01 à 1% en poids du poids total de la composition de rinçage intermédiaire.

10. Procédé selon l'une quelconque des revendications précédentes **caractérisé en ce que** la composition de rinçage intermédiaire comprend au moins un polymère cationique, anionique, non-ionique ou amphotère, et/ou au moins une silicone.

11. Procédé selon l'une quelconque des revendications précédentes **caractérisé en ce que** la composition de rinçage intermédiaire ne contient pas de bétaïne.

12. Procédé selon l'une quelconque des revendications précédentes **caractérisé en ce que** la composition de rinçage intermédiaire ne contient pas de tensioactif amphotère.

13. Procédé selon l'une quelconque des revendications précédentes **caractérisé en ce que** le pH de la composition de rinçage intermédiaire est compris entre 2 et 7, de préférence entre 3 et 5.

14. Procédé selon l'une quelconque des revendications précédentes **caractérisé en ce que** la composition oxydante comprend au moins un agent oxydant choisi parmi le peroxyde d'hydrogène, les bromates alcalins, les polythionates, les persels, tels que les perborates, les percarbonates et les persulfates.

15. Procédé selon la revendication 14 **caractérisé en ce que** le ou les agents oxydants représentent généralement de 0,1 à 8%, de préférence de 0,2 à 5%, en poids par rapport au poids total de la composition oxydante.

16. Procédé selon l'une quelconque des revendications précédentes **caractérisé en ce que** le pH de la composition oxydante varie de 1 à 13, de préférence de 1,5 à 8, mieux de 1,5 à 5.

17. Procédé selon l'une quelconque des revendications précédentes **caractérisé en ce que** on laisse la composition de rinçage intermédiaire agir pendant 30 secondes à 30 minutes.

18. Procédé selon l'une quelconque des revendications précédentes **caractérisé en ce que** l'étape d'application de la composition réductrice et/ou l'étape d'application de la composition de rinçage intermédiaire et/ou l'étape de fixation s'effectue(nt) sous chauffage ou est (sont) immédiatement suivie(s) d'un chauffage.

19. Procédé selon l'une quelconque des revendications précédentes **caractérisé en ce qu'**il comprend, après l'étape de fixation par oxydation, une étape de rinçage des fibres kératiniques à l'eau et/ou au moyen d'un reliquat de la composition de rinçage intermédiaire.

## Patentansprüche

1. Verfahren zur permanenten Verformung von Keratinfasern, insbesondere des Haars, umfassend:
- einen Schritt des Aufbringens einer reduzierenden Zusammensetzung auf die Keratinfasern zur Reduktion der Disulfidbindungen des Keratins, wobei die Keratinfasern vor, während oder nach dem Aufbringen, vorzugsweise vorher, unter mechanische Spannung gesetzt werden, dann
- einen Schritt des Aufbringens einer Zwischenspülungszusammensetzung, umfassend:
- mindestens ein einwertiges Metallkationsalz und/oder mindestens ein Ammoniumsalz, wobei das Salz bzw. die Salze aus NaCl, NH₄Cl, KC1 und AgCl ausgewählt ist bzw. sind, und
- mindestens eine organische Säure,
auf die Keratinfasern, wobei es sich bei dem Salz bzw. den Salzen nicht um ein Salz bzw. Salze der organischen Säure bzw. der organischen Säuren, das bzw. die in der Zwischenspülungszusammensetzung vorliegt bzw. vorliegen, handelt und es sich bei dem Salz bzw. den Salzen nicht um ein Tensid bzw. Tenside handelt, dann
- einen Schritt des oxidativen Fixierens zur Wiederherstellung der Bindungen durch Aufbringen einer oxidierenden Zusammensetzung auf die Keratinfasern,
wobei das Verfahren keinen Schritt des Spülens mit Wasser zwischen dem Schritt des Aufbringens der reduzierenden Zusammensetzung und dem Schritt des Aufbringens der Zwischenspülungszusammensetzung oder zwischen dem Schritt des Aufbringens der Zwischenspülungszusammensetzung und dem Schritt des Aufbringens der oxidierenden Zusammensetzung umfasst.

2. Verfahren nach Anspruch 1, **dadurch gekennzeichnet, dass** man die reduzierende Zusammensetzung über einen Zeitraum von 5 bis 60 Minuten und vorzugsweise über einen Zeitraum von 5 bis 30 Minuten einwirken lässt.

3. Verfahren nach Anspruch 1 oder 2, **dadurch gekennzeichnet, dass** die reduzierende Zusammensetzung in einem kosmetisch unbedenklichen Medium mindestens ein Reduktionsmittel umfasst, das aus den Reduktionsmitteln der Formel
H(X')_{q}(R')ᵣ
wobei X' für P, S oder SO₂ steht, q gleich 0 oder 1 ist, r gleich 1 oder 2 ist und R' für einen linearen, verzweigten, gesättigten, ungesättigten (C₁-C₂₀)Kohlenwasserstoffrest, der gegebenenfalls durch ein Heteroatom unterbrochen ist und gegebenenfalls Substituenten, die aus einer Hydroxygruppe, einer halogenierten Gruppe, einer Amingruppe oder einer Carboxygruppe, einer ((C₁-C₃₀)Alkoxy)carbonylgruppe, einer Amidogruppe, einer ((C₁-C₃₀)Alkyl)aminocarbonylgruppe, einer ((C₁-C₃₀)-Acyl)aminogruppe, einer Monoalkylamino- oder Dialkylaminogruppe, einer Monohydroxylamino- oder Dihydroxylaminogruppe ausgewählt sind, umfasst, steht, oder einem Salz davon in Kombination mit einer Base ausgewählt ist.

4. Verfahren nach Anspruch 3, **dadurch gekennzeichnet, dass** das Reduktionsmittel bzw. die Reduktionsmittel aus Thioglykolsäure, Thiomilchsäure, Glycerinmonothioglycolat, Cysteamin, N-Acetylcysteamin, N-Propionylcysteamin, Cystein, N-Acetylcystein, Thioäpfelsäure, Panthetein, 2,3-Dimercaptobernsteinsäure, N-(Mercaptoalkyl)-ω-hydroxyalkylamiden, N-Mono- oder N,N-Dialkyl-mercapto-4-butyramiden, Aminomercaptoalkylamiden, Derivaten von N-(Mercaptoalkyl)succinamidsäuren und N-(Mercaptoalkyl)succinimiden, Alkylaminomercaptoalkylamiden, dem azeotropen Gemisch von 2-Hydroxypropylthioglykonat und (2-Hydroxy-1-methyl)ethylthioglykolat, Mercaptoalkylaminoamiden, N-Mercaptoalkylalkandiamiden und Formamidinsulfinsäurederivaten und Salzen davon ausgewählt ist bzw. sind.

5. Verfahren nach Anspruch 3 oder 4, **dadurch gekennzeichnet, dass** das Reduktionsmittel bzw. die Reduktionsmittel 0,05 bis 30 Gew.-%, vorzugsweise 1 bis 20 Gew.-% und noch besser 4 bis 11 Gew.-%, bezogen auf das Gesamtgewicht der reduzierenden Zusammensetzung, ausmacht bzw. ausmachen.

6. Verfahren nach einem der vorhergehenden Ansprüche, **dadurch gekennzeichnet, dass** das Salz bzw. die Salze 0,1 bis 20 Gew.-%, vorzugsweise 0,1 bis 10 Gew.-% und noch besser 0,5 bis 8 Gew.-%, bezogen auf das Gesamtgewicht der Zwischenspülungszusammensetzung, ausmacht bzw. ausmachen.

7. Verfahren nach einem der vorhergehenden Ansprüche, **dadurch gekennzeichnet, dass** die organische Säure bzw. die organischen Säuren aus gesättigten oder ungesättigten Säuren mit einer oder mehreren Carbonsäure-, Sulfonsäure-, Phosphonsäure- oder Phosphorsäuregruppen ausgewählt ist bzw. sind.

8. Verfahren nach Anspruch 7, **dadurch gekennzeichnet, dass** die organische Säure bzw. die organischen Säuren aus Essigsäure, Propansäure, Butansäure, Milchsäure, Glykolsäure, Ascorbinsäure, Phthalsäure, Maleinsäure, Bernsteinsäure und Citronensäure, vorzugsweise Citronensäure, ausgewählt ist bzw. sind.

9. Verfahren nach einem der vorhergehenden Ansprüche, **dadurch gekennzeichnet, dass** die organische Säure bzw. die organischen Säuren 0,005 bis 10 Gew.-% und vorzugsweise 0,01 bis 1 Gew.-%, bezogen auf das Gesamtgewicht der Zwischenspülungszusammensetzung, ausmacht bzw. ausmachen.

10. Verfahren nach einem der vorhergehenden Ansprüche, **dadurch gekennzeichnet, dass** die Zwischenspülungszusammensetzung mindestens ein kationisches, anionisches, nichtionisches oder amphoteres Polymer und/oder mindestens ein Silikon umfasst.

11. Verfahren nach einem der vorhergehenden Ansprüche, **dadurch gekennzeichnet, dass** die Zwischenspülungszusammensetzung kein Betain enthält.

12. Verfahren nach einem der vorhergehenden Ansprüche, **dadurch gekennzeichnet, dass** die Zwischenspülungszusammensetzung kein amphoteres Tensid enthält.

13. Verfahren nach einem der vorhergehenden Ansprüche, **dadurch gekennzeichnet, dass** der pH-Wert der Zwischenspülungszusammensetzung zwischen 2 und 7 und vorzugsweise zwischen 3 und 5 liegt.

14. Verfahren nach einem der vorhergehenden Ansprüche, **dadurch gekennzeichnet, dass** die oxidierende Zusammensetzung mindestens ein Oxidationsmittel umfasst, das aus Wasserstoffperoxid, Alkalimetallbromaten, Polythionaten und Persalzen, wie Perboraten, Percarbonaten und Persulfaten, ausgewählt ist.

15. Verfahren nach Anspruch 14, **dadurch gekennzeichnet, dass** das Oxidationsmittel bzw. die Oxidationsmittel im Allgemeinen 0,1 bis 8 Gew.-% und vorzugsweise 0,2 bis 5 Gew.-%, bezogen auf das Gesamtgewicht der oxidierenden Zusammensetzung, ausmacht bzw. ausmachen.

16. Verfahren nach einem der vorhergehenden Ansprüche, **dadurch gekennzeichnet, dass** der pH-Wert der oxidierenden Zusammensetzung im Bereich von 1 bis 13, vorzugsweise von 1,5 bis 8 und noch besser von 1,5 bis 5 liegt.

17. Verfahren nach einem der vorhergehenden Ansprüche, **dadurch gekennzeichnet, dass** man die Zwischenspülungszusammensetzung über einen Zeitraum von 30 Sekunden bis 30 Minuten einwirken lässt.

18. Verfahren nach einem der vorhergehenden Ansprüche, **dadurch gekennzeichnet, dass** der Schritt des Aufbringens der reduzierenden Zusammensetzung und/oder der Schritt des Aufbringens der Zwischenspülungszusammensetzung und/oder der Fixierungsschritt unter Erhitzen erfolgt bzw. erfolgen oder unmittelbar danach erhitzt wird.

19. Verfahren nach einem der vorhergehenden Ansprüche, **dadurch gekennzeichnet, dass** es nach dem Schritt der oxidativen Fixierung einen Schritt des Spülens der Keratinfasern mit Wasser und/oder mit einer Restmenge der Zwischenspülungszusammensetzung umfasst.

## Claims

1. Method for permanently deforming keratin fibres, in particular hair, comprising:
- a step of applying a reducing composition to the keratin fibres, to reduce the disulfide bonds in the keratin, the keratin fibres being placed under mechanical tension before, during or after said application, preferably before, then
- a step of applying an intermediate rinsing composition to the keratin fibres, comprising:
- at least one monovalent metal cation salt and/or at least one ammonium salt, the salt(s) being chosen from NaCl, NH₄Cl, KCl and AgCl, and
- at least one organic acid,
said salt(s) not being one or more salt(s) of said organic acid(s) present in the intermediate rinsing composition, and said salt(s) not being one or more surfactant(s), then
- a step of fixing by oxidation, to reform said bonds, by applying an oxidizing composition to the keratin fibres,
said process not comprising a step of rinsing with water between the step of applying the reducing composition and the step of applying the intermediate rinsing composition, nor between the step of applying the intermediate rinsing composition and the step of applying the oxidizing composition.

2. Process according to Claim 1, **characterized in that** the reducing composition is left to act for 5 to 60 minutes, preferably for 5 to 30 minutes.

3. Process according to Claim 1 or 2, **characterized in that** the reducing composition comprises, in a cosmetically acceptable medium, at least one reducing agent chosen from the reducing agents of formula
H(X')_{q}(R')ᵣ
in which X' represents P, S or SO₂, q is equal to 0 or 1, r is equal to 1 or 2 and R' is a linear or branched, saturated or unsaturated, (C₁-C₂₀) hydrocarbon-based radical optionally interrupted by a heteroatom, and optionally comprising substituents chosen from a hydroxy group, a halogenated group, an amine group or a carboxy group, a ((C₁-C₃₀)alkoxy)carbonyl group, an amido group, a ((C₁-C₃₀)alkyl)aminocarbonyl group, a ((C₁-C₃₀)acyl)amino group, a monoalkylamino or dialkylamino group, a monohydroxylamino or dihydroxylamino group,
or a salt thereof in combination with a base.

4. Process according to Claim 3, **characterized in that** the reducing agent(s) are chosen from thioglycolic acid, thiolactic acid, glyceryl monothioglycolate, cysteamine, N-acetylcysteamine, N-propionylcysteamine, cysteine, N-acetylcysteine, thiomalic acid, pantetheine, 2,3-dimercaptosuccinic acid, N-(mercaptoalkyl)-ω-hydroxyalkylamides, N-monoalkylmercapto-4-butyramides or N,N-dialkylmercapto-4-butyramides, aminomercaptoalkylamides, derivatives of N- (mercaptoalkyl)succinamic acids and of N-(mercaptoalkyl)succinimides, alkylaminomercaptoalkylamides, the azeotropic mixture of 2-hydroxypropyl thioglyconate and of (2-hydroxy-1-methyl)ethyl thioglycolate, mercaptoalkylaminoamides, N-mercaptoalkylalkanediamides and formamidine sulfinic acid derivatives and salts thereof.

5. Process according to Claim 3 or 4, **characterized in that** the reducing agent (s) represent from 0.05 to 30%, preferably from 1 to 20%, better still from 4 to 11% by weight relative to the total weight of the reducing composition.

6. Process according to any one of the preceding claims, **characterized in that** the salt(s) represent from 0.1 to 20%, preferably from 0.1 to 10%, better still from 0.5 to 8% by weight relative to the total weight of the intermediate rinsing composition.

7. Process according to any one of the preceding claims, **characterized in that** the organic acid(s) are chosen from saturated or unsaturated acids comprising one or more carboxylic, sulfonic, phosphonic or phosphoric acid functions.

8. Process according to Claim 7, **characterized in that** the organic acid(s) are chosen from acetic acid, propanoic acid, butanoic acid, lactic acid, glycolic acid, ascorbic acid, phthalic acid, maleic acid, succinic acid and citric acid, preferably citric acid.

9. Process according to any one of the preceding claims, **characterized in that** the organic acid(s) represent from 0.005 to 10%, preferably from 0.01 to 1% by weight of the total weight of the intermediate rinsing composition.

10. Process according to any one of the preceding claims, **characterized in that** the intermediate rinsing composition comprises at least one cationic, anionic, nonionic or amphoteric polymer and/or at least one silicone.

11. Process according to any one of the preceding claims, **characterized in that** the intermediate rinsing composition does not contain betaine.

12. Process according to any one of the preceding claims, **characterized in that** the intermediate rinsing composition does not contain amphoteric surfactant.

13. Process according to any one of the preceding claims, **characterized in that** the pH of the intermediate rinsing composition is between 2 and 7, preferably between 3 and 5.

14. Process according to any one of the preceding claims, **characterized in that** the oxidizing composition comprises at least one oxidizing agent chosen from hydrogen peroxide, alkali metal bromates, polythionates, persalts such as perborates, percarbonates and persulfates.

15. Process according to Claim 14, **characterized in that** the oxidizing agent(s) generally represent from 0.1 to 8%, preferably from 0.2 to 5% by weight relative to the total weight of the oxidizing composition.

16. Process according to any one of the preceding claims, **characterized in that** the pH of the oxidizing composition ranges from 1 to 13, preferably from 1.5 to 8, better still from 1.5 to 5.

17. Process according to any one of the preceding claims, **characterized in that** the intermediate rinsing composition is left to act for 30 seconds to 30 minutes.

18. Process according to any one of the preceding claims, **characterized in that** the step of applying the reducing composition and/or the step of applying the intermediate rinsing composition and/or the fixing step is (are) carried out with heating or is (are) immediately followed by heating.

19. Process according to any one of the preceding claims, **characterized in that** it comprises, after the step of fixing by oxidation, a step of rinsing the keratin fibres with water and/or by means of the remains of the intermediate rinsing composition.
